# FASCICULE DE BREVET EUROPEEN

(11) **EP 0 691 839 B1**
(45) Date de publication et mention de la délivrance du brevet: **04.12.1996**
(21) Numéro de dépôt: 94911985.3
(22) Date de dépôt: 28.03.1994
(51) Int. Cl.: A61K 7/42, A61K 7/00

(54) **EMULSION A BASE DE DIHYDROXYACETONE ET SON UTILISATION EN COSMETIQUE**
EMULSION AUF DER BASIS VON DIHYDROXYACETON UND IHRE VERWENDUNG IN KOSMETIK
DIHYDROXYACETONE-BASED EMULSION AND ITS USE IN COSMETICS

(30) Priorité: 29.03.1993 FR 9303600
(43) Date de publication de la demande: 17.01.1996
(73) Titulaire: L'OREAL, 75008 Paris (FR)
(72) Inventeur: HANSENNE, Isabelle, F-75017 Paris (FR)
(74) Mandataire: Casalonga, Axel
(86) Numéro de dépôt international: FR9400341
(87) Numéro de publication internationale: WO9422418

(56) Documents cités:
- EP-A- 0 328 099
- EP-A- 0 500 446
- DE-A- 1 141 049
- DE-A- 1 926 359
- DE-A- 2 722 725
- FR-A- 1 293 290

## Description

L'invention est relative à une émulsion à base de dihydroxyacétone ou DHA et au procédé de coloration de la peau mettant en oeuvre une telle émulsion.

La dihydroxyacétone ou DHA est couramment utilisée comme agent de bronzage artificiel de la peau. Pour être appliquée uniformément sur la peau, la DHA est de préférence formulée sous forme d'émulsion telle qu'une crème ou un lait.

Cependant, les formulations sous forme d'émulsion posent des problèmes de conservation au stockage, notamment de stabilité et d'évolution de la couleur dans le temps. C'est ainsi que l'on a pu observer une instabilité de l'émulsion au cours du temps, notamment lorsqu'on la porte à 45°C, et une évolution de la coloration vers les jaunes et les ambrés, notamment après six semaines de stockage à 45°C.

En outre, une telle émulsion étant appliquée sur la peau, on cherche à réduire le plus possible la concentration en émulsionnants qui peuvent être irritants. Une telle émulsion doit en outre présenter de bonnes propriétés cosmétiques, c'est-à-dire être facile à étaler, ne pas coller après application et présenter une bonne résistance à l'eau.

Les émulsions à base de DHA proposées jusqu'a maintenant posent généralement des problèmes de conservation au bout de quelques jours ou quelques semaines à 45°C, prennent une coloration jaune ou ambrée non souhaitable ou encore présentent l'inconvénient de coller après application.

Le brevet DE-A-2722725 décrit des compositions cosmétiques conférant à la peau une coloration brun-rouge, à base d'un composé diéthylénique en association avec la dihydroxyacétone. Les émulsionnants utilisés dans ces compositions sont des émulsionnants usuels.

Le brevet FR-A-1 293 290 décrit des préparations cosmétiques stables sous forme de crèmes contenant de la dihydroxyécatone en association avec un filtre solaire exempt de groupes aminés. L'émulsionnant utilisé est un mélange de stéarate de polyéthylène sorbitan et de sesquioléate de sorbitan.

Le brevet DE-A-1 926 359 décrit une crème anti-solaire comprenant de la dihydroxyacétone en association avec un filtre solaire et des corps gras usuels. L'émulsionnant utilisé est un mélange de monostéarate de sorbitol et de monostéarate de sorbitol polyoxyéthyléné.

La demanderesse a découvert qu'en utilisant dans une émulsion eau-dans-huile contenant de la DHA dans la phase aqueuse, un émulsionnant bien particulier, dans des proportions spécifiques, introduit dans la phase grasse de l'émulsion comprenant des proportions spécifiques d'isoparaffine ou de poly-α-oléfine et de silicone et éventuellement de corps gras additionnels, on obtenait une composition de coloration de la peau sous forme d'émulsion eau-dans l'huile stable au stockage, présentant un faible jaunissement dans le temps, non irritante, présentant une bonne résistance à l'eau et de bonnes propriétés cosmétiques dans la mesure où elle est facile à étaler et ne colle pas après application. En outre, cette émulsion communique à la peau une coloration esthétiquement améliorée.

L'invention a donc pour objet une émulsion cosmétique stable de coloration de la peau, du type eau-dans-huile, constituée :
**A**) de 50 à 92% en poids, sur la base du poids total de l'émulsion, d'une phase aqueuse comprenant 0,5 à 10% en poids de dihydroxyacétone, et
**B**) de 8 à 50% en poids, sur la base du poids total de l'émulsion, d'une phase grasse comprenant 5 à 15% en poids d'isoparaffine ou de poly-α-oléfine, et 3 à 7% en poids d'un émulsionnant constitué d'un mélange d'esters de sorbitol et de glycérol d'acides gras en C₁₈ à chaîne droite ou ramifiée, saturée ou insaturée, comportant éventuellement un radical hydroxyle, ces esters étant oxyéthylénés et oxypropylénés, le nombre total d'unités d'oxyde d'éthylène et d'oxyde de propylène étant inférieur ou égal à 5,
   tous les pourcentages étant donnés sur la base du poids total de l'émulsion.

L'émulsion selon l'invention peut également contenir dans la phase grasse, jusqu'à 10% en poids de silicone et/ou jusqu'à 15% en poids, sur la base du poids total de l'émulsion, de corps gras additionnels tels que les acides gras en C₈-C₂₂, les alcools gras, par exemple les alcools laurylique, cétylique, myristique, stéarylique, palmitique, oléique ainsi que le 2-octyldodécanol, les esters d'acides gras tels que le monostéarate de glycérol et le monostéarate de polyéthylèneglycol et les triglycérides d'acides gras en C₆-C₁₈ tels que les triglycérides d'acide caprylique/caprique (Miglyol 812).

La dihydroxyacétone ou DHA est présente dans l'émulsion eau-dans-huile selon l'invention dans des proportions suffisantes pour conférer à la peau, après application, une coloration similaire à la coloration obtenue a la suite d'un bronzage naturel. Elle est généralement présente dans des proportions comprises entre 0,5 et 10% en poids par rapport au poids total de l'émulsion, et de préférence comprises entre 1 et 7% en poids.

Un émulsionnant préféré selon l'invention est le dérivé polyoxyéthyléné (2,5 moles d'oxyde d'éthylène) et polyoxypropyléné (1,5 mole d'oxyde de propylène), d'un mélange d'esters du glycérol et du sorbitol des acides hydroxystéarique et isostéarique, vendu sous la dénomination "ARLACEL 780" par la Société ICI.

L'émulsion cosmétique eau-dans-huile selon l'invention peut être utilisée pour réaliser une émulsion triple E/H/E, c'est-à-dire eau-dans-huile-dans-eau. Dans une telle émulsion triple, le pourcentage d'émulsion primaire E/H varie de 55 à 80% en poids par rapport au poids total de l'émulsion triple. La stabilité de cette émulsion triple est assurée en ajoutant un émulsionnant du type copolymère en bloc polyoxyde d'éthylène/polyoxyde de propylène dans des proportions de 0,75 à 2% en poids rapport au poids total de l'émulsion triple.

Ce copolymère en bloc est solubilisé dans la phase aqueuse externe continue de l'émulsion triple. Si nécessaire, la phase aqueuse externe peut contenir un épaississant tel qu'un dérivé de cellulose.

L'invention a donc également pour objet l'utilisation de l'émulsion E/H ci-dessus pour la préparation d'une émulsion triple E/H/E.

Un autre objet de l'invention est constitué par une émulsion cosmétique stable de coloration de la peau, du type émulsion triple E/H/E, contenant 55 à 80% en poids de l'émulsion E/H ci-dessus, dans 45 à 20% en poids, sur la base du poids total de l'émulsion triple, d'une phase aqueuse externe continue contenant 0,75 à 2% en poids par rapport au poids total de l'émulsion triple, d'un émulsionnant du type copolymère en bloc polyoxyde d'éthylène/polyoxyde de propylène, ainsi qu'éventuellement un épaississant.

L'invention a encore pour objet le procédé de coloration de la peau mettant en oeuvre une émulsion cosmétique E/H telle que définie ci-dessus ou l'émulsion cosmétique triple E/H/E obtenue à partir de cette dernière.

L'isoparaffine ou la poly-α-oléfine utilisée dans la phase grasse de l'émulsion cosmétique selon l'invention appartient aux groupes de composés suivants :
- les isoparaffines de viscosité dynamique inférieure à 0,5 Pa.s répondant à la formule : 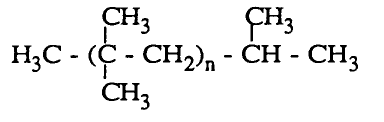 dans laquelle n est compris entre 2 et 16 et leurs mélanges avec des huiles de structure identique, dans laquelle n est supérieur à 18 et de préférence compris entre 18 et 40; de telles huiles sont vendues par la Société PRESPERSE INC. sous les dénominations "PERMETHYL 99A, 101A, 102A, 104A, 106A",
   ou par la Société ICI sous la dénomination "ARLAMOL HD";
   on peut citer, aussi les "ISOPARS" vendus par la Société EXXON INC;
- les poly-α-oléfines de type polydécène, hydrogénées ou non; de tels produits sont vendus par la Société ETHYL CORPORATION sous la dénomination "ETHYLFLO"; on cite aussi les polyisobutylènes hydrogénés ou non.

Lorsque l'émulsion contient des silicones, celles-ci sont des organopolysiloxanes tels que les huiles d'organopolysiloxanes, des solutions organiques de gommes et/ou de résines d'organosiloxanes.

Parmi ces silicones, on peut citer les silicones volatiles ayant un point d'ébullition compris entre 60°C et 260°C, telles que les silicones cycliques comportant de 3 à 7 atomes de silicium, les silicones volatiles linéaires ayant de 2 à 9 atomes de silicium et ayant une viscosité cinématique inférieure ou égale à 5 x 10⁻⁶m²/s à 25°C.

Les silicones non volatiles sont constituées principalement par les polyalkylsiloxanes, les polyarylsiloxanes, les polyalkylarylsiloxanes, les copolymères de polyéthersiloxanes modifiés ou non, les gommes et résines de silicone et les polysiloxanes organo-modifiés, ainsi que leurs mélanges.

Parmi les polyalkylsiloxanes, on peut citer :
- les polydiméthylsiloxanes linéaires de viscosité cinématique supérieure à 10⁻¹ m²/s, pouvant comporter des groupements terminaux triméthylsilyle, trihydroxysilyle ou hydroxydiméthylsilyle;
- les polyalkylarylsiloxanes, tels que les polydiméthylphénylsiloxanes, les polyméthyldiphénylsiloxanes linéaires et/ou ramifiés de viscosité cinématique de 10⁻⁵ à 5.10⁻²m²/s à 25°C;
- les polyalkyl (C₁₀-C₃₀) siloxanes à groupements terminaux triméthylsilyle;
- les copolymères de polyéthersiloxanes modifiés ou non sont choisis notamment parmi les copolymères d'oxyde d'éthylène et/ou de propylène avec un diorganosiloxane, tels que les diméthicone copolyols;
- les gommes de silicone sont entre autres des polydiorganosiloxanes de masse moléculaire comprise entre 200 000 et 1 000 000, utilisés seuls ou en mélange, dans un solvant choisi parmi les silicones volatiles, les huiles de polydiméthylsiloxanes, les huiles de polyphénylméthylsiloxanes, les isoparaffines, le dodécane, le tridécane, le tétradécane ou leurs mélanges;
- les résines d'organo-polysiloxanes utilisables conformément à l'invention sont des systèmes siloxaniques réticulés renfermant des unités R₂ SiO_{2/2}, R Si O_{3/2}, Si O_{4/2} dans lesquelles R représente un groupement hydrocarboné possédant de 1 à 6 atomes de carbone ou un groupement phényle; le groupement hydrocarboné désigne de préférence un radical alkyle en C₁-C₄;
- les silicones organo-modifiées sont choisies notamment parmi les silicones précitées comportant dans leur structure générale un ou plusieurs groupements organofonctionnels directement fixés sur la chaîne siloxanique ou fixés par l'intermédiaire d'un radical hydrocarboné. On peut citer à cet effet les silicones comportant des groupements perfluorés tels que des groupements trifluoroalkyle, des groupements hydroxylés comme les polyorganosiloxanes à fonction hydroxyalkyle, des groupements alcoxylés, des groupements acyloxyalkyle.

Les polyorganosiloxanes particulièrement préférés sont choisis parmi :
- les silicones non volatiles du type polyalkylsiloxanes linéaires à groupements terminaux triméthylsilyle comme les huiles "SILBIONE" des séries 70 047 et 47 telles que l'huile 47V 500 000, commercialisées par la Société RHONE POULENC, ou l'huile "DC 200 FLUID" de la Société DOW CORNING;
- les mélanges d'organopolysiloxanes et de silicones cycliques volatiles tels que les produits Q2 1 401 ou Q2 3225C de la Société DOW CORNING ou SF 1214 SILICONE FLUID de la Société GENERAL ELECTRIC;
- les diméthicone copolyols tels que le produit Q2 5220 de la Société DOW CORNING;
- les fluorosilicones de type polyalkylsiloxanes à groupements terminaux triméthylsilyle et substitués sur la chaîne par des groupements trifluoropropyle tels que la fluorosilicone vendue par la Société SHIN ETSU sous la dénomination "X 22-821";
- les silicones volatiles linéaires ou cycliques et plus particulièrement le décaméthylcyclopentasiloxane et ses mélanges, par exemple les produits "DC 245 FLUID" et "DC 345 FLUID" de la Société DOW CORNING.

Selon une forme de réalisation préférée, l'émulsion cosmétique selon l'invention contient des filtres solaires liposolubles ou hydrosolubles UV-B et/ou UV-A. Elle peut aussi contenir des polymères filtres et/ou des silicones filtres, tels que ceux décrits dans la demande de brevet français n° 2 680 683.

Les filtres solaires sont choisis en particulier parmi les dérivés cinnamiques tels que par exemple le p-méthoxycinnamate de 2-éthylhexyle; les dérivés salicyliques comme par exemple le salicylate de 2-éthylhexyle; les dérivés du camphre comme par exemple le 4-méthylbenzylidène camphre, l'acide benzène 1,4[di(3-méthylidène 10-camphosulfonique)]; les dérivés du benzimidazole tels que l'acide 2-phénylbenzimidazole 5-sulfonique; les dérivés de triazine tels que la 2,4,6-tris [p-(2'-éthylhexyl-1'-oxycarbonyl)anilino] 1,3,5-triazine; les dérivés de la benzophénone tels que la 2-hydroxy 4-méthoxy benzophénone; les dérivés du dibenzoylméthane tels que le 4-tert-butyl 4'-méthoxydibenzoylméthane, les dérivés de β,β,-diphénylacrylate tels que le α-cyano-β,β-diphénylacrylate de 2-éthylhexyle.

Ces filtres solaires sont utilisés dans des proportions comprises entre 0,01 et 15% en poids par rapport au poids total de l'émulsion.

Outre l'eau, l'émulsion cosmétique selon l'invention peut contenir à titre de solvants jusqu'à 5% en poids, sur la base du poids total de l'émulsion, d'un ou plusieurs alkylèneglycols, par exemple l'éthylèneglycol, le propylèneglycol et le diéthylèneglycol.

L'émulsion conforme à l'invention peut également contenir tout autre adjuvant utilisé habituellement dans les compositions cosmétiques pour la peau et plus particulièrement des agents hydratants tels que des polyols, par exemple la glycérine ou le sorbitol, des pigments et nanopigments d'oxydes métalliques, des agents adoucissants, des vitamines, des antioxydants, des agents de stabilisation de l'émulsion primaire, par exemple le chlorure de sodium, le chlorure de magnésium, le sulfate de magnésium ou le produit dénommé "ELFACOS C 26" vendu par la Société AKZO CHEMIE, qui est un ester de l'alcool hydroxyoctacosanylique et de l'acide hydroxystéarique, ainsi que des parfums et des conservateurs.

Les solvants, corps gras ou adjuvants utilisés dans les émulsions conformes à l'invention ne doivent comporter ni groupement amine primaire ou secondaire, ni groupement oxydant susceptible d'interférer avec la DHA.

L'émulsion selon l'invention peut également contenir ou être utilisée conjointement avec des substances pouvant avoir un effet de nuançage ou de coloration supplémentaire de la peau en association avec la DHA. On peut utiliser à cet effet en particulier des hydroxyindoles, des pigments mélaniques d'origine naturelle ou synthétique.

Le procédé de coloration de la peau qui est un autre objet de l'invention est essentiellement caractérisé par le fait que l'on applique sur la peau, une émulsion telle que définie ci-dessus dans des quantités suffisantes pour conférer à la peau une coloration similaire au bronzage naturel.

Les exemples qui suivent sont destinés à illustrer l'invention sans pour autant présenter un caractère limitatif.

### Exemple 1 - Emulsion E/H

| | | |
|---|---|---|
| - *Emulsionnant* : Dérivé polyoxyéthyléné (2,5 moles d'oxyde d'éthylène) et polyoxypropyléné (1,5 mole d'oxyde de propylène), d'un mélange d'esters du glycérol et du sorbitol des acides hydroxystéarique et isostéarique, vendu sous la dénomination "ARLACEL 780" par la Société ICI | | 3,0 g |
| - DHA | | 4,0 g |
| - *Stabilisants* : | sulfate de magnésium 7 H₂O | 0,7 g |
| | "ELFACOS C₂₆" d'AKZO CHEMIE | 3,0 g |
| - *Poly-α-oléfine* : Polydécène hydrogéné vendu sous la dénomination "ETHYLFLO 362 NF" par la Société ETHYL CORPORATION | | 7,0 g |
| - Diméthicone copolyol vendu sous la dénomination "Q₂ 5220" par la Société DOW CORNING | | 5,0 g |
| - Conservateurs, parfums qs | | |
| -Eau qsp | | 100 g |

La phase aqueuse est chauffée à 70°C. La phase grasse est également chauffée à 70°C.

On réalise l'émulsion en ajoutant la phase aqueuse à la phase grasse sous vive agitation au Moritz. Lorsque la température de l'émulsion descend vers 40°C, on ajoute la DHA solubilisée au préalable dans environ 10 g d'eau. On poursuit l'agitation. On ajoute enfin la silicone puis les conservateurs et parfums. On maintient l'agitation jusqu'à refroidissement complet de l'émulsion.

### Exemple 2 - Emulsion E/H

| | | |
|---|---|---|
| - *Emulsionnant* : "ARLACEL 780" | | 3,0 g |
| - DHA | | 4,0 g |
| - *Stabilisants* : | sulfate de magnésium 7 H₂O | 0,7 g |
| | "ELFACOS C₂₆" d'AKZO CHEMIE | 3,0 g |
| - *Poly-α-oléfine* : Polydécène hydrogéné vendu sous la dénomination "ETHYLFLO 362 NF" par la Société ETHYL CORPORATION | | 7,0 g |
| - Conservateurs, parfums qs | | |
| - Eau qsp | | 100 g |

### Exemple 3 - Emulsion E/H

| | | |
|---|---|---|
| - *Emulsionnant* : "ARLACEL 780" | | 4,0 g |
| - DHA | | 6,0 g |
| - *Stabilisants* : | sulfate de magnésium 7 H₂O | 0,7 g |
| | "ELFACOS C₂₆" d'AKZO CHEMIE | 3,0 g |
| - *Isoparaffine* : Isododécane vendu sous la dénomination "PERMETHYL 99A" par la Société PRESPERSE INC | | 9,0 g |
| - Décaméthylcyclopentasiloxane vendu sous la dénomination "DC 245 FLUID" par la Société DOW CORNING | | 7,0 g |
| - Conservateurs, parfums qs | | |
| - Eau qsp | | 100 g |

### Exemple 4 - Emulsion E/H

| | | |
|---|---|---|
| - *Emulsionnant* : "ARLACEL 780" | | 6,0 g |
| - DHA | | 2,5 g |
| - *Stabilisants* : | sulfate de magnésium 7 H₂O | 0,7 g |
| | "ELFACOS C₂₆" d'AKZO CHEMIE | 3,0 g |
| - 2- décanoxyundécanol vendu sous la dénomination "EXXAL 21" par la Société EXXON | | 6,0 g |
| - Mélange de diméthiconol (13%), d'octaméthylcyclotétrasiloxane et de décaméthylcyclopentasiloxane (87%) vendu sous la dénomination "Q₂-1401" par la Société DOW CORNING | | 4,0 g |
| - *Poly-α-oléfine* : Polydécène hydrogéné vendu sous la dénomination "ETHYLFLO 168" par la Société ETHYL CORPORATION | | 6,0 g |
| - Conservateurs, parfums qs | | |
| - Eau qsp | | 100 g |

### Exemple 5 - Emulsion triple E/H/E

### A - Emulsion primaire E/H

| | |
|---|---|
| - *Emulsionnant* : "ARLACEL 780" | 3,0 g |
| - DHA | 5,0 g |
| - *Isoparaffine* : Heptaméthylnonane vendu sous la dénomination "ARLAMOL HD" par la Société ICI | 5,0 g |
| - Polydiméthylsiloxane de viscosité 10⁻² m²/s vendu sous la dénomination "DC 200 FLUID" par la Société DOW CORNING | 5,0 g |
| - Glycérine | 3,0 g |
| - Conservateurs, parfums qs | |
| - Eau qsp | 60 g |

### B - Emulsion triple E/H/E

| | |
|---|---|
| - Copolymère en bloc OE/OP (Poloxamer 407) vendu par la société ICI sous la dénomination "SYMPERONIC PE/F 127" | 1,0 g |
| - Glycérine | 2,0 g |
| - Hydroxyéthylcellulose vendue sous la dénomination "NATROSOL 250 HHR" par la Société AQUALON | 0,3 g |
| - Conservateurs, parfums qs | |
| - Eau qsp | 100 g |

On prépare l'émulsion primaire E/H. On porte l'émulsionnant secondaire constitué par le "SYMPERONIC PE/F127" à 80°C dans 18 g d'eau. On prépare un gel avec le "NATROSOL 250 HHR" dans le restant d'eau. A 20°C, on ajoute le gel de "NATROSOL" à la solution de "SYMPERONIC PE/F127", puis glycérine, conservateur et parfums. On ajoute ensuite l'émulsion primaire dans la phase aqueuse gélifiée. On agite jusqu'à obtention de l'émulsion E/H/E.

### Exemple 6 - Emulsion E/H

| | | |
|---|---|---|
| - *Emulsionnant* : "ARLACEL 780" | | 5,0 g |
| - *Isoparaffine* : Heptaméthylnonane vendu sous la dénomination "ARLAMOL HD" par la Société ICI | | 10,0 g |
| - *Stabilisants* : | sulfate de magnésium 7 H₂O | 0,7 g |
| | "ELFACOS C₂₆" d'AKZO CHEMIE | 3,0 g |
| - Mélange de silicones cycliques : décaméthylcyclopentasiloxane/octaméthylcyclotétrasiloxane/dodécaméthylcyclohexasiloxane vendu sous la dénomination "DC 345 FLUID" par la Société DOW CORNING (62 à 72/4/24) | | 5,0 g |
| - Mélange de polydiméthylsiloxane à chaînes latérales polyoxyéthylénées ou polyoxypropylénées (diméthicone copolyol) et d'un polydiméthylsiloxane cyclique, vendu sous la dénomination "Q₂-3225" par la Société DOW CORNING | | 5,0 g |
| - Glycérine | | 3,0 g |
| - DHA | | 5,0 g |
| - Conservateurs, parfums qs | | |
| - Eau qsp | | 100 g |

### Exemple 7 - Emulsion E/H

| | | |
|---|---|---|
| - *Emulsionnant* : "ARLACEL 780" | | 5,0 g |
| - *Isoparaffine* : "ARLAMOL HD" | | 15,0 g |
| - Mélange de silicones cycliques "DC 345 FLUID" de DOW CORNING | | 10,0 g |
| - *Stabilisants* : | sulfate de magnésium 7 H₂O | 0,7 g |
| | "ELFACOS C₂₆" d'AKZO CHEMIE | 3,0 g |
| - DHA | | 5,0 g |
| - Conservateurs, parfums qs | | |
| - Eau qsp | | 100 g |

## Revendications

1. Emulsion cosmétique stable de coloration de la peau, de type E/H caractérisée par le fait qu'elle comprend :
**A**) 50 à 92% en poids, sur la base du poids total de l'émulsion, d'une phase aqueuse comprenant 0,5 à 10% en poids de dihydroxyacétone, sur la base du poids total de l'émulsion, et
**B**) 8 à 50% en poids, sur la base du poids total de l'émulsion, d'une phase grasse comprenant 5 à 15% en poids d'isoparaffine ou de poly-α-oléfine et 3 à 7% en poids d'un émulsionnant constitué d'un mélange d'esters de sorbitol et de glycérol d'acides gras en C₁₈ à chaîne droite ou ramifiée, saturée ou insaturée, comportant éventuellement un hydroxyle, ces esters étant oxyéthylénés et oxypropylénés, le nombre total d'unités d'oxyde d'éthylène et d'oxyde de propylène étant inférieur ou égal à 5, tous les pourcentages étant donnés sur la base du poids total de l'émulsion.

2. Emulsion cosmétique selon la revendication 1, caractérisée par le fait que la dihydroxyacétone est utilisée en des proportions comprises entre 1 et 7% en poids, par rapport au poids total de l'émulsion.

3. Emulsion cosmétique selon la revendication 1 ou 2, caractérisée par le fait qu'elle contient en outre jusqu'à 10% en poids de silicone.

4. Emulsion cosmétique selon l'une quelconque des revendications 1 à 3, caractérisée par le fait qu'elle contient en outre jusqu'à 15% en poids de corps gras choisis parmi les acides gras en C₈-C₂₂, les alcools gras, les esters d'acides gras, et les triglycérides d'acides gras en C₆-C₁₈.

5. Emulsion cosmétique selon l'une quelconque des revendications 1 à 4, caractérisée par le fait que l'isoparaffine est choisie parmi les isoparaffines de viscosité dynamique inférieure à 0,5 Pa.s répondant à la formule : 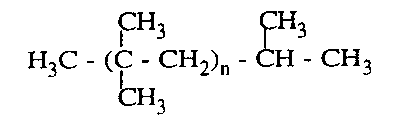 dans laquelle n est compris entre 2 et 16 et leurs mélanges avec des huiles de structure identique dans laquelle n est supérieur à 18 et de préférence compris entre 18 et 40.

6. Emulsion cosmétique selon l'une quelconque des revendications 1 à 4, caractérisée par le fait que la poly-α-oléfine est choisie parmi les poly-α-oléfines de type polydécène, hydrogénées ou non hydrogénées, et les polyisobutylènes hydrogénés ou non hydrogénés.

7. Emulsion cosmétique selon l'une quelconque des revendications 3 à 6, caractérisée par le fait que la silicone est choisie parmi les silicones volatiles cycliques ou linéaires, ayant un point d'ébullition compris entre 60 et 260°C, les silicones non volatiles constituées par les polyalkylsiloxanes, les polyarylsiloxanes, les polyalkyarylsiloxanes, les copolymères de polyéthersiloxanes modifiés ou non, les gommes et résines de silicone et les polysiloxanes organo-modifiés, ainsi que leurs mélanges.

8. Emulsion cosmétique selon l'une quelconque des revendications 1 à 7, caractérisée par le fait que l'émulsionnant est un dérivé polyoxyéthyléné à 2,5 moles d'oxyde d'éthylène et polyoxypropyléné à 1,5 mole d'oxyde de propylène d'un mélange d'esters du glycérol et du sorbitol des acides hydroxy stéarique et isostéarique.

9. Emulsion cosmétique selon l'une quelconque des revendications 1 à 8, caractérisée par le fait qu'elle contient également des filtres solaires liposolubles ou hydrosolubles UV-B ou UV-A.

10. Emulsion cosmétique selon la revendication 9, caractérisée par le fait que les filtres solaires sont choisis parmi les cinnamates, les salicylates, les dérivés du benzylidène camphre, les dérivés de benzimidazole, les dérivès de triazine, les dérivés de la benzophénone, les dérivés du dibenzoylméthane, les dérivés de β,β-diphénylacrylate, les polymères filtres et les silicones filtres.

11. Emulsion cosmétique selon l'une quelconque des revendications 1 à 10, caractérisée par le fait qu'elle contient des adjuvants cosmétiquement acceptables choisis parmi les solvants du type alkylèneglycols, les hydratants du type polyols, les adoucissants, les vitamines, les antioxydants, les agents de stabilisation de l'émulsion primaire, les parfums et les conservateurs, les pigments et nanopigments d'oxydes métalliques.

12. Utilisation de l'émulsion cosmétique E/H selon l'une quelconque des revendications 1 à 11 pour la réalisation d'une émulsion cosmétique triple E/H/E.

13. Emulsion cosmétique triple du type E/H/E, caractérisée par le fait qu'elle comprend 55 à 80% en poids, sur la base du poids total de l'émulsion triple, d'émulsion primaire E/H selon l'une quelconque des revendications 1 à 11 dans 45 à 20% en poids, sur la base du poids total de l'émulsion triple, d'une phase aqueuse continue externe contenant 0,75 à 2% en poids, sur la base du poids total de l'émulsion triple, d'un émulsionnant du type copolymère en bloc polyoxyde d'éthylène/polyoxyde de propylène, ainsi qu'éventuellement un épaississant.

14. Procédé cosmétique de coloration de la peau pour lui conférer un bronzage similaire au bronzage naturel, caractérisé par le fait que l'on applique sur la peau une émulsion de type E/H ou E/H/E telle que définie dans l'une quelconque des revendications 1 à 11 ou 13.

## Claims

1. Stable cosmetic emulsion for colouring the skin, of W/O type, characterized in that it comprises:
**A**) 50 to 92% by weight, on the basis of the total weight of emulsion, of an aqueous phase comprising 0.5 to 10% by weight of dihydroxyacetone, on the basis of the total weight of the emulsion [sic], and
**B**) 8 to 50% by weight, on the basis of the total weight of the emulsion, of a fatty phase comprising 5 to 15% by weight of isoparaffin or of poly-α-olefin and 3 to 7% by weight of an emulsifier consisting of a mixture of sorbitol and of glycerol esters of saturated or unsaturated, straight- or branched-chain C₁₈ fatty acids, optionally containing a hydroxyl, these esters being oxyethylenated and oxypropylenated, the total number of ethylene oxide and of propylene oxide units being less than or equal to 5, all the percentages being given on the basis of the total weight of the emulsion.

2. Cosmetic emulsion according to Claim 1, characterized in that dihydroxyacetone is used in proportions of between 1 and 7% by weight with respect to the total weight of the emulsion.

3. Cosmetic emulsion according to Claim 1 or 2, characterized in that it additionally contains up to 10% by weight of silicone.

4. Cosmetic emulsion according to any one of Claims 1 to 3, characterized in that it additionally contains up to 15% by weight of fatty substances chosen from C₈-C₂₂ fatty acids, fatty alcohols, fatty acid esters and triglycerides of C₆-C₁₈ fatty acids.

5. Cosmetic emulsion according to any one of Claims 1 to 4, characterized in that the isoparaffin is chosen from isoparaffins with a dynamic viscosity of less than 0.5 Pa·s corresponding to the formula: 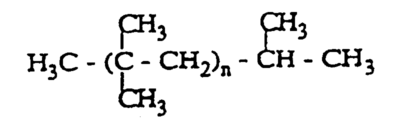 in which n is between 2 and 16, and their mixtures with oils of identical structure in which n is greater than 18 and preferably between 18 and 40.

6. Cosmetic emulsion according to any one of Claims 1 to 4, characterized in that the poly-α-olefin is chosen from poly-α-olefins of polydecene type, which are hydrogenated or are not hydrogenated, and polyisobutylene, which are hydrogenated or are not hydrogenated.

7. Cosmetic emulsion according to any one of Claims 3 to 6, characterized in that the silicone is chosen from cyclic or linear volatile silicones having a boiling point of between 60 and 260°C and non-volatile silicones composed of polyalkylsiloxanes, polyarylsiloxanes, polyalkylarylsiloxanes, copolymers of polyethersiloxanes which may or may not be modified, silicone gums and resins and organomodified siloxanes, and their mixtures.

8. Cosmetic emulsion according to any one of Claims 1 to 7, characterized in that the emulsifier is a polyoxyethylenated, containing 2.5 mol of ethylene oxide, and polyoxypropylenated, containing 1.5 mol of propylene oxide, derivative of a mixture of the glycerol and of the sorbitol esters of hydroxy [sic] stearic and isostearic acids.

9. Cosmetic emulsion according to any one of Claims 1 to 8, characterized in that it also contains fat-soluble or water- soluble UV-B or UV-A sunscreens.

10. Cosmetic emulsion according to Claim 9, characterized in that the sunscreens are chosen from cinnamates, salicylates, benzylidene camphor derivatives, benzimidazole derivates, triazine derivatives, benzophenone derivatives, dibenzoylmethane derivatives, β,β-diphenylacrylate derivatives, polymer screening agents and silicone screening agents.

11. Cosmetic emulsion according to any one of Claims 1 to 10, characterized in that it contains cosmetically acceptable adjuvants chosen from solvents of the alkylene glycol type, humectants of the polyol type, emollients, vitamins, antioxidants, agents for stabilizing the primary emulsion, fragrances and preservatives, and metal oxide pigments and nanopigments.

12. Use of the W/O cosmetic emulsion according to any one of Claims 1 to 11 in the preparation of a triple W/O/W cosmetic emulsion.

13. Triple cosmetic emulsion of the W/O/W type, characterized in that it comprises 55 to 80% by weight, on the basis of the total weight of the triple emulsion, of primary W/O emulsion according to any one of Claims 1 to 11 in 45 to 20% by weight, on the basis of the total weight of the triple emulsion, of a continuous external aqueous phase containing 0.75 to 2% by weight, on the basis of the total weight of the triple emulsion, of an emulsifier of the poly(ethylene oxide)/poly(propylene oxide) block copolymer type and optionally a thickener.

14. Cosmetic process for colouring the skin in order to confer on it a tanning similar to natural tanning, characterized in that an emulsion of W/O or W/O/W type as defined in any one of Claims 1 to 11 or 13 is applied to the skin.

## Patentansprüche

1. Stabile kosmetische W/O-Emulsion zur Färbung der Haut,
dadurch **gekennzeichnet**, daß
sie enthält:
A) 50 bis 92 Gew.%, bezogen auf das Gesamtgewicht der Emulsion, einer wässrigen Phase, die 0,5 bis 10 Gew.% Dihydroxyaceton enthält, sowie
B) 8 bis 50 Gew.%, bezogen auf das Gesamtgewicht der Emulsion, einer Fett-Phase, die 5 bis 15 Gew.% Isoparaffin oder Poly-α-olefin und 3 bis 7 Gew.% eines Emulgators aus einer Mischung aus Sorbitol- und Glycerylestern von geraden oder verzweigten, gesättigten oder ungesättigten, gegebenenfalls einen Hydroxylrest aufweisenden C₁₈-Fettsäuren enthält, wobei diese Ester oxethyliert und oxpropyliert sind, und wobei die Gesamtzahl an Ethylenoxid- und Propylenoxid-Einheiten weniger als oder gleich 5 beträgt,
wobei alle Prozentsätze auf Basis des Gesamtgewichts der Emulsion angegeben sind.

2. Kosmetische Emulsion gemäß Anspruch 1,
dadurch **gekennzeichnet**, daß
das Dihydroxyaceton in Mengenanteilen von 1 bis 7 Gew.% verwendet wird, bezogen auf das Gesamtgewicht der Emulsion.

3. Kosmetische Emulsion gemäß Anspruch 1 oder 2,
dadurch **gekennzeichnet**, daß
sie außerdem bis zu 10 Gew.% Silikon enthält.

4. Kosmetische Emulsion gemäß einem der Ansprüche 1 bis 3,
dadurch **gekennzeichnet**, daß
sie außerdem bis zu 15 Gew.% Fettkörper enthält, ausgewählt aus C₈₋₂₂-Fettsäuren, Fettalkoholen, Fettsäureestern und aus Triglyceriden von C₆₋₁₈-Fettsäuren.

5. Kosmetische Emulsion gemäß einem der Ansprüche 1 bis 4,
dadurch **gekennzeichnet**, daß
das Isoparaffin aus Isoparaffinen einer dynamischen Viskosität von weniger als 0,5 Pa x s der Formel: 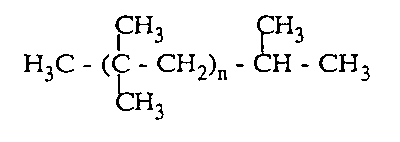 worin n 2 bis 16 beträgt, sowie aus deren Mischungen mit Ölen identischer Struktur, worin n mehr als 18 und vorzugsweise 18 bis 40 beträgt, ausgewählt ist.

6. Kosmetische Emulsion gemäß einem der Ansprüche 1 bis 4,
dadurch **gekennzeichnet**, daß
das Poly-α-olefin aus Poly-α-olefinen vom Typ Polydecen, hydriert oder nicht hydriert, und aus hydrierten oder nicht hydrierten Polyisobutylenen ausgewählt ist.

7. Kosmetische Emulsion gemäß einem der Ansprüche 3 bis 6,
dadurch **gekennzeichnet**, daß
das Silikon aus zyklischen oder linearen flüchtigen Silikonen mit einem Siedepunkt von 60 bis 260°C, aus nicht-flüchtigen Silikonen aus Polyalkylsiloxanen, Polyarylsiloxanen, Polyalkylarylsiloxanen, gegebenenfalls modifizierten Polyethersiloxan-Copolymeren, Gummi- und Harzprodukten aus Silikon und organomodifizierten Polysiloxanen sowie aus deren Mischungen ausgewählt ist.

8. Kosmetische Emulsion gemäß einem der Ansprüche 1 bis 7,
dadurch **gekennzeichnet**, daß
der Emulgator ein mit 2,5 Mol Ethylenoxid polyoxethyliertes und mit 1,5 Mol Propylenoxid polyoxpropyliertes Derivat einer Mischung aus Glyceryl- und Sorbitolestern von Hydroxystearin- und Isostearinsäuren ist.

9. Kosmetische Emulsion gemäß einem der Ansprüche 1 bis 8,
dadurch **gekennzeichnet**, daß
sie auch fett- oder wasserlösliche UV-B- oder UV-A-Sonnenfilterstoffe enthält.

10. Kosmetische Emulsion gemäß Anspruch 9,
dadurch **gekennzeichnet**, daß
die Sonnenfilterstoffe aus Cinnamaten, Salicylaten, Benzylidenkampferderivaten, Benzimidazolderivaten, Triazinderivaten, Derivaten des Benzophenons, Derivaten des Dibenzoylmethans, Derivaten von β,β-Diphenylacrylat, polymeren Filterstoffen und aus Filterstoffen aus Silikonen ausgewählt sind.

11. Kosmetische Emulsion gemäß einem der Ansprüche 1 bis 10,
dadurch **gekennzeichnet**, daß
sie kosmetisch geeignete Hilfsstoffe enthält, ausgewählt aus Lösungsmittel vom Typ der Alkylenglycole, aus hydratisierenden Mitteln vom Typ der Polyole, aus Weichmachern, Vitaminen, Antioxidantien, Stabilisiermitteln für die Primäremulsion, Parfüm-Produkten und Konservierungsmitteln sowie aus Pigmenten und Nanopigmenten aus Metalloxiden.

12. Verwendung der kosmetischen W/O-Emulsion gemäß einem der Ansprüche 1 bis 11 zur Herstellung einer kosmetischen W/O/W-Dreifachemulsion.

13. Kosmetische Dreifachemulsion vom W/O/W-Typ,
dadurch **gekennzeichnet**, daß
sie 55 bis 80 Gew.%, bezogen auf das Gesamtgewicht der Dreifachemulsion, W/O-Primäremulsion gemäß einem der Ansprüche 1 bis 11 in 45 bis 20 Gew.%, bezogen auf das Gesamtgewicht der Dreifachemulsion, einer äußeren kontinuierlichen wässrigen Phase enthält, die wiederum 0,75 bis 2 Gew.%, bezogen auf das Gesamtgewicht der Dreifachemulsion, eines Emulgators vom Typ eines Block-Copolymer aus Ethylenpolyoxid/Propylenpolyoxid sowie gegebenenfalls ein Verdickungsmittel enthält.

14. Kosmetisches Verfahren zur Färbung der Haut, um ihr eine der natürlichen Bräunung ähnliche Bräunung zu verleihen,
dadurch **gekennzeichnet**, daß
man auf die Haut eine in jedem der Ansprüche 1 bis 11 definierte Emulsion vom W/O-Typ oder eine in Anspruch 13 definierte Emulsion vom W/O/W-Typ aufträgt.
